(19) Europäisches Patentamt
European Patent Office
Office européen des brevets

(11) **EP 2 574 337 B1**

(12) **EUROPEAN PATENT SPECIFICATION**

(45) Date of publication and mention
of the grant of the patent:
**09.05.2018  Bulletin 2018/19**

(51) Int Cl.:
*A61K 31/01* (2006.01)   *A61K 9/107* (2006.01)
*A61K 9/14* (2006.01)    *A61K 47/14* (2017.01)
*A61K 47/22* (2006.01)   *A61K 47/36* (2006.01)
*A61P 3/02* (2006.01)    *A61K 8/31* (2006.01)
*A61K 8/37* (2006.01)    *A61Q 19/00* (2006.01)
*A61Q 19/02* (2006.01)   *A61K 8/06* (2006.01)

(21) Application number: **11783593.4**

(22) Date of filing: **18.05.2011**

(86) International application number:
**PCT/JP2011/061451**

(87) International publication number:
**WO 2011/145659 (24.11.2011 Gazette 2011/47)**

(54) **CAROTENOID-CONTAINING COMPOSITION AND PRODUCTION METHOD THEREFOR**

KAROTINOIDHALTIGE ZUSAMMENSETZUNG UND HERSTELLUNGSVERFAHREN DAFÜR

COMPOSITION À BASE DE CAROTÉNOÏDE ET SON PROCÉDÉ DE PRODUCTION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.03.2011  JP 2011072918
18.02.2011  JP 2011033812
18.05.2010  JP 2010114793**

(43) Date of publication of application:
**03.04.2013  Bulletin 2013/14**

(73) Proprietor: **FUJIFILM Corporation
Tokyo 106-8620 (JP)**

(72) Inventor: **SERIZAWA, Shinichiro
Ashigarakami-gun
Kanagawa 258-8577 (JP)**

(74) Representative: **Hoffmann Eitle
Patent- und Rechtsanwälte PartmbB
Arabellastraße 30
81925 München (DE)**

(56) References cited:
**WO-A1-2009/093595    JP-A- 2007 269 749
JP-A- 2008 143 841**

- **DATABASE WPI Week 200225 Thomson
Scientific, London, GB; AN 2002-191334
XP002744873, & JP 2001 316601 A (FUJI KAGAKU
KOGYO KK) 16 November 2001 (2001-11-16)**
- **DATABASE WPI Week 201038 Thomson
Scientific, London, GB; AN 2010-F09695
XP002744874, & JP 2010 105972 A (NIPPON
MENARD KESHOHIN KK) 13 May 2010
(2010-05-13) & DATABASE WPI Week 200859
Thomson Scientific, London, GB; AN
2008-K00887 & JP 2008 074758 A (NOEVIR KK) 3
April 2008 (2008-04-03)**
- **DATABASE WPI Week 200308 Thomson
Scientific, London, GB; AN 2003-084861
XP002744875, & JP 2002 326922 A (KOSE KK) 15
November 2002 (2002-11-15)**
- **DATABASE WPI Week 200801 Thomson
Scientific, London, GB; AN 2008-A05759
XP002744876, & JP 2007 269749 A (FUJI FILM CO
LTD) 18 October 2007 (2007-10-18)**
- **DATABASE WPI Week 200971 Thomson
Scientific, London, GB; AN 2009-P75671
XP002744877, & JP 2009 234933 A (PICASSO
BIKAGAKU KENKYUSHO KK) 15 October 2009
(2009-10-15)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The present invention relates to a carotenoid-containing composition and a method of producing the same.

Background Art

Recently, various compositions containing a carotenoid are proposed focusing attention on a high functionality of the carotenoid. Generally since the carotenoid is widely known as a poorly-soluble material, the form of emulsified composition of the carotenoid is usually employed.
As an emulsified composition containing a carotenoid, specifically there are known compositions such as a carotenoid-containing emulsion composition in which at least one water-soluble emulsifying agent is contained in an aqueous phase and tocopherol and lecithin are contained in an oil phase (Japanese Patent Application Laid-Open (JP-A) No. 2008-13751); a food carotenoid type dye solubilized solution formulation in which a composition of the food carotenoid type dye and poly glycerin fatty acid ester is microparticulated and when its absorbance at the maximum absorption wavelength of the visible portion is 1, transmittance at 660 nm is 99% or more (JP-ANo. 10-120933); a carotenoid-containing composition formed by emulsifying an oil phase in which carotenoids have been dissolved in fat and oil, in a polyalcohol-containing aqueous phase in the presence of polyglycerine fatty acid ester and lecithin, in which an average particle diameter of the oil phase is 100 nm or less(JP-ANo. 9-157159); and the like.

SUNMMARY OF INVENTION

Problem to be Solved by Invention

However, a crystalline carotenoid such as lycopene exhibits high crystallinity, and for example, a crystalline body often remains at the time of preparation of an emulsified composition. In the composition containing such crystalline body, sometimes expected effects are not obtained due to the presence of the crystalline body.
Accordingly, a first aspect of the present invention aims to provide a method of producing a carotenoid-containing composition in which crystallization is suppressed, even though the composition contains a crystalline carotenoid.
Further, a second aspect of the present invention aims to provide a carotenoid-containing composition in which a crystalline carotenoid can be stably contained in the non-crystalline state.

Means for Solving Problem

The present invention is as follows.

[1] A method of producing a carotenoid-containing composition, the method comprising:

obtaining a carotenoid-containing oil phase composition by heating an oil phase component mixed liquid comprising a carotenoid component comprising at least lycopene, and diglyceryl monostearate , at a temperature of the melting point of the carotenoid component or higher; and
obtaining an oil-in-water emulsified composition by emulsifying under pressure the carotenoid-containing oil phase composition with an aqueous-phase composition comprising an emulsifying agent;

wherein the lycopene content is from 0.1 % by mass to 5% by mass with respect to the total mass of the solid content in the carotenoid-containing composition;
wherein the total mass of diglyceryl monostearate is from 0.01 times to 10 times with respect to the total mass of lycopene;
the method further comprising incorporating an antioxidant in the oil phase component mixed liquid before the heating, wherein the antioxidant is at least one selected from the group consisting of ascorbic acid, an ascorbic acid ester, and a salt thereof.
[2] The method of producing a carotenoid-containing composition according to [1], wherein a total mass of the antioxidant is from 0.05 times to 50 times with respect to a total mass of the crystalline carotenoid.
[3] The method of producing a carotenoid-containing composition according to any one of [1] to [2], wherein the carotenoid-containing composition further comprises at least one water-soluble encapsulating agent selected from a sugar polymer comprising a sugar unit comprising at least two fructose units or an oligomer comprising a sugar unit comprising at least two fructose units.

[4] The method of producing a carotenoid-containing composition according to [3], wherein a total mass of the water-soluble encapsulating agent is from 0.5 times to 50 times with respect to a total mass of oil components comprising the carotenoid component.

[5] The method of producing a carotenoid-containing composition according to any one of [1] to [4], wherein a total mass of the emulsifying agent contained in the aqueous phase composition is from 0.1 times to 10 times with respect to a total mass of oil components comprising the carotenoid component.

[6] The method of producing a carotenoid-containing composition according to any one of [1] to [5], further comprising obtaining a powder composition by drying the oil-in-water emulsified composition.

[7] The method of producing a carotenoid-containing composition according to any one of [1] to [6], wherein an average particle diameter of a re-dissolved oil-in-water emulsified composition that has been obtained by re-dissolving the oil-in-water emulsified composition or the powder composition is from 50 nm to 300 nm.

[8] A carotenoid-containing composition obtained by the method as defined in [1], comprising: a carotenoid component comprising at least lycopene, at least 90% by mass of the lycopene being non-crystalline; and diglyceryl monostearate;

wherein the lycopene content is from 0.1 % by mass to 5% by mass with respect to the total mass of the of the solid content in the carotenoid-containing composition; and wherein the total mass of diglyceryl monostearate is from 0.01 times to 10 times with respect to the total mass of lycopene.

Effect of Invention

According to a first aspect of the present invention, there is provided a method of producing a carotenoid-containing composition in which crystallization is suppressed, even though the composition contains a crystalline carotenoid, i.e. lycopene.

Further, according to a second aspect of the present invention, there is provided a carotenoid-containing composition in which a crystalline carotenoid, i.e. lycopene, can be stably contained in the non-crystalline state.

BEST EMBODIMENT FOR CARRYING OUT INVENTION

A method of producing a carotenoid-containing composition of the present invention includes obtaining a carotenoid-containing oil phase composition by heating an oil phase component mixed liquid containing a carotenoid component comprising at least lycopene, and diglyceryl monostearate, at a temperature of the melting point of the carotenoid component or higher (hereinafter referred to as "a process of preparing a carotenoid-containing oil phase composition"); and obtaining an oil-in-water emulsified composition by emulsifying under pressure the carotenoid-containing oil phase composition with an aqueous-phase composition containing an emulsifying agent (hereinafter referred to as "a process of preparing an oil-in-water emulsified composition"), as defined in claim 1.

According to the production method of the present invention, since a carotenoid component comprising at least lycopene is heated together with diglyceryl monostearate under temperature conditions at the melting point of the carotenoid component or higher, the carotenoid component is co-dissolved with diglyceryl monostearate. By using a carotenoid-containing oil phase composition that is obtained by the above-described co-dissolving as the oil phase composition to be emulsified by heat together with an aqueous composition containing an aqueous emulsifying agent, the carotenoid-containing composition obtained by the emulsification is a composition in which crystallization of the crystalline carotenoid is suppressed.

The carotenoid-containing composition of the present invention includes a carotenoid component containing at least lycopene, at least 90% by mass of the lycopene being non-crystalline; and diglyceryl monostearate. The carotenoid-containing composition of the present invention is obtained by the production method of the present invention, as defined in claim 1.

In the present specification, any numerical range expressed herein using "to" refers to a range including the numerical values before and after "to" as the minimum and maximum values, respectively.

In a case in which the amount of a component in the composition is indicated in the invention, when there are plural substances corresponding to the component in the composition, the indicated amount means the total amount of the plural substances present in the composition, unless specifically stated otherwise.

Hereinafter, descriptions are given about the present invention.

[Carotenoid-containing oil phase composition]

In the production method of the present invention, the carotenoid-containing oil phase composition obtained in the process of preparing a carotenoid-containing oil phase composition is prepared from an oil phase component mixed liquid containing a carotenoid component containing at least lycopene, and diglyceryl monostearate.

The "crystalline carotenoid" in the present invention refers to lycopene.

The crystalline carotenoid is not limited to naturally-derived materials, but also may be any materials, as long as it is obtained in accordance with an ordinary method. Further, identity of the crystalline carotenoid may be confirmed in the usual manner. For example, confirmation can be conducted by differential scanning calorimetry (DSC), observation under a polarizing microscope, X-ray diffraction, or the like.

Lycopene is a compound exhibiting high antioxidant effectiveness and white effectiveness, and its addition to foods, cosmetics, raw materials for pharmaceutical products and processed product are desired, considered and practiced.

Lycopene is a carotenoid having chemical formula $C_{40}H_{56}$ (molecular weight: 536.87) and belongs to carotenes that is one of the carotenoid. Lycopene is a red-color colorant having an absorption maximum at 474 nm (acetone).

In lycopene, there are also cis- trans isomers with respect to a conjugated double bond at the center of the molecule. Examples of the isomers include all-trans isomer, 9-cis isomer and 13-cis isomer. In the present invention, any of these isomers may be used.

In the carotenoid-containing composition obtained in the present invention, lycopene may be contained in the form of a lycopene-containing oil or a lycopene-containing paste that has been separated or extracted from natural products containing the lycopene.

In nature, lycopene is contained in tomato, persimmon, watermelon and pink grapefruit, and the above-described lycopene-containing oil may be those separated or extracted from these natural products.

Further, lycopene used in the present invention may be the above-described extracted material, or if need, an appropriately purified substance, or alternatively a synthetic compound.

In the present invention, it is especially preferable from the viewpoints of quality and productivity that lycopene is a tomato extract.

Further in the present invention, a widely-marketed tomato extract may be used as the lycopene-containing oil or lycopene-containing paste. Examples thereof include Lyc-O-Mato 15% and Lyc-O-Mato 6%, commercially-available from SUNBRIGHT CO., LTD. and LYCOPENE 18, commercially-available from Kyowa Hakko Kirin Co., Ltd.

The crystalline carotenoid may constitutes a carotenoid component as a simple body, or alternatively together with an oil content (oil) that is used in the case when carotenoid is extracted from a natural product.

The lycopene content is from 0.1% by mass to 5% by mass, preferably from 0.2% by mass to 4% by mass, and more preferably from 0.3% by mass to 3% by mass, with respect to a total mass of the solid content (all components excluding water) in the carotenoid-containing composition. If the lycopene content is within the above-described range, effects due to the crystalline carotenoid can be expected.

In the present invention, diglyceryl monostearate is used, which is a specific (poly)glyceryl fatty acid ester that forms an oil phase mixed liquid with a carotenoid component.

Such a specific (poly)glyceryl fatty acid ester exhibits high compatibility with a crystalline carotenoid, and in the case of constituting a carotenoid-containing oil phase composition as a co-melting material, the specific (poly)glyceryl fatty acid ester suppresses crystallization of the crystalline carotenoid.

The content (mass) of diglyceryl monostearate is from 0.01 times to 10 times, preferably from 0.1 times to 8 times, and more preferably from 0.3 times to 5 times, with respect to a total mass of lycopene from the viewpoint of stability of the carotenoid-containing composition. If the total mass of diglyceryl monostearate in the carotenoid-containing composition 0.01 times with respect to a total mass of lycopene, a sufficient crystallization suppression effect can be expected. Meanwhile, if the total mass of diglyceryl monostearate is 10 times or less, it is possible to suppress increase in the particle diameter of emulsion particles in case of an emulsion is formed.

In order to suppress decomposition of the lycopene, the oil phase component mixed liquid contains an antioxidant as one of other components that are other than lycopene and diglyceryl monostearate. Such antioxidant is at least one material selected from ascorbic acid, ascorbic acid ester or their salts. The use of the above-described antioxidant (hereinafter, sometimes referred to as "ascorbic acid-based antioxidant") enables to suppress with a certainty decomposition of lycopene by heat (for example, oxidation decomposition and the like), thereby suppressing reduction in lycopene during the production process of a carotenoid-containing composition.

Examples of the ascorbic acid-based antioxidant include L-ascorbic acid, sodium L-ascorbate, potassium L-ascorbate, calcium L-ascorbate, L-ascorbic acid-phosphate, L-ascorbic acid-phosphate magnesium salt, L-ascorbic acid-sulfate, L-ascorbic acid-sulfate 2-sodium salt, L-ascorbyl stearate, L-ascorbyl 2-glycoside, L-ascorbyl palmitate, and L-ascorbyl tetraisopalmitate; and ascorbic fatty acid esters such as L-ascorbyl stearate, L-ascorbyl tetraisopalmitate and L-ascorbyl palmitate. Among these, L-ascorbic acid, sodium L-ascorbate, calcium L-ascorbate, L-ascorbyl stearate, L-ascorbyl 2-glycoside, L-ascorbyl palmitate, L-ascorbic acid-phosphate magnesium salt, L-ascorbic acid-sulfate 2-sodium salt and L-ascorbyl tetraisopalmitate are especially preferred from the viewpoint of reduction in temperature loss of carotenoid.

These ascorbic acid-based antioxidants may be contained alone in an oil phase mixed liquid, or may be compounded in the oil phase mixed liquid in the form of its aqueous solution. Although the concentration of the ascorbic acid-based antioxidant in the aqueous solution is not particularly limited, the range of from 0.05% by mass to 5% by mass is generally preferred from the viewpoint of oxidation prevention.

The total mass of the ascorbic acid-based antioxidant contained in the oil phase mixed liquid is preferably from 0.05 times to 50 times, more preferably from 1 time to 10 times, still more preferably from 1.5 times to 10 times, and still more preferably from 2 times to 10 times, with respect to the mass of the crystalline carotenoid from the viewpoint of reduction in thermal loss of a carotenoid. If the mass of the ascorbic acid-based antioxidant is at least 0.05 times with respect to the crystalline carotenoid, it is enough to exert an inhibitory effect on reduction in the content of the crystalline carotenoid. Meanwhile, if the mass of the ascorbic acid-based antioxidant is at most 50 times, it does not impair compounding of a sufficient amount of the crystalline carotenoid.

The oil phase composition (carotenoid-containing oil phase composition) is obtained by subjecting a mixed liquid of the above-described oil phase components (oil phase component- mixed liquid) to a heat treatment.

As for the temperature at the time of heating of the oil phase component- mixed liquid, it is necessary to be at least a melting point of the carotenoid component. When the temperature is lower than the melting point, a crystalline carotenoid does not dissolve, so that a great amount of crystalline body becomes present in the carotenoid-containing composition.

The melting point of the carotenoid component means a temperature at which a crystalline carotenoid in the carotenoid component dissolves. In the case where the carotenoid component is constituted by lycopene alone, a melting point of the crystalline carotenoid corresponds to the melting point of lycopene. Meanwhile, in the case where components other than lycopene are contained in the carotenoid component, a melting point of the carotenoid component means a temperature at which lycopene in the carotenoid component dissolves. For example, it is known that in the case where lycopene-containing oil derived from a natural substance is used as a carotenoid component, sometimes impurities may be contained therein, so that lycopene in the carotenoid component dissolves at a temperature lower than the melting point of the crystalline carotenoid. In this case, the temperature at which the lycopene in the carotenoid component dissolves corresponds to the "melting point of the carotenoid component" in the present invention. The melting point of the carotenoid component may be confirmed in accordance with a method generally used to confirm a melting point. For example, the melting point may be confirmed in accordance with DSC.

The heat temperature to be applied to prepare a lycopene-containing oil phase composition varies depending on, in specific terms, the carotenoid components to be used. Generally, however, the heat temperature may be within a range of from 150°C to 200°C, and from the viewpoint of suppressing thermal deposition, preferably from 150°C to 180°C, and more preferably from 150°C to 170°C.

As for the maximum heat temperature to be applied to prepare a carotenoid-containing oil phase composition, from the viewpoint of suppressing deposition of a crystalline carotenoid, the highest temperature in a heat treatment is preferably a temperature such that a difference from a melting point of the carotenoid component is within 10°C. A temperature slightly exceeding the melting point, for example, a temperature within 5°C from the melting point is more preferred.

The heat time is optional, as long as it is a time required for dissolving a carotenoid component in the oil phase component- mixed liquid. From the viewpoint of effectively suppressing both amophization of a crystalline body and decomposition of a carotenoid due to excessive heat, the heat time is preferably from 10 minutes to 60 minutes and more preferably from 15 minutes to 45 minutes. However, the heat time is not limited thereto.

By such heat treatment, a carotenoid-containing oil phase composition can be obtained from an oil phase component mixed liquid containing both lycopene and diglyceryl monostearate.

Further, it is important to perform the heat treatment so that the oil phase component mixed liquid in its entirety becomes uniform temperature. Accordingly, sufficient agitation while heating is preferred and it is desirable for the oil phase component mixed liquid to be maintained at a definite temperature by using an airtight container and heating while stirring.

In addition to the each component described above, the carotenoid-containing oil phase composition may contain other oil components that are ordinarily used as an oil phase component.

The other oil component is not particularly limited, as long as it does not dissolve in an aqueous medium, but dissolves in an oil medium. They can be used by appropriate selection of a material having desired properties and functions for different purposes. For example, non-crystalline carotenoids, unsaturated fatty acids, oils and fats such as coconut oil, fat-soluble vitamins such as tocopherol, and ubiquinones are preferably used.

Examples of the unsaturated fatty acids include univalent highly-unsaturated fatty acids having at least 10 carbon atoms and preferably 18 to 30carbon atoms ($\omega$-9, oleic acid and the like) and polyvalent highly-unsaturated fatty acids having at least 10 carbon atoms and preferably 18 to 30carbon atoms (co-3, co-6). These unsaturated fatty acids may be any of known ones, and examples thereof include linolenic acid, eicosapentaenoic acid (EPA) and docosahexaenoic acid (DHA), and fish oils containing these materials.

Examples of the ubiquinones include Coenzyme Q types such as Coenzyme Q-10.

Examples of the fat-soluble vitamins include fat-soluble vitamin E types, vitamin A types, vitamin D types and oil-soluble derivatives of erythorbic acid. Among these, fat-soluble vitamin E types that exhibit high anti-oxidation function and also can be used as a radical scavenger are preferred.

The vitamin E types are not particularly limited. Examples thereof include those selected from the group consisting of tocopherol and derivatives thereof and the group consisting of tocotrienol and derivatives thereof. These may be used singly or in combination of two or more kinds. Further, any of those selected from the group consisting of tocopherol and derivatives thereof and any of those selected from the group consisting of tocotrienol and derivatives thereof may be used in combination.

Examples of the group consisting of tocopherol and derivatives thereof include dl-$\alpha$-tocopherol, dl-$\beta$-tocoberol, dl-$\gamma$-tocopherol, dl-$\delta$-tocopherol, dl-$\alpha$-tocopherol acetate, dl-$\alpha$-tocopherol nicotinate, dl-$\alpha$-tocopherol linoleate, and dl-$\alpha$-tocopherol succinate. Among these, dl-$\alpha$-tocopherol, dl-$\beta$-tocopherol, dl-$\gamma$-tocopherol, dl-$\delta$-tocopherol, and these mixtures (mixed tocopherol) are more preferable. Further, as for the tocopherol derivatives, carboxylic acid esters of tocopherol, especially acetic acid esters thereof are preferably used.

Examples of the group consisting of tocotrienol and derivatives thereof include $\alpha$-tocotrienol, $\beta$-tocotrienol, $\gamma$-tocopherol and $\delta$-tocopherol. Further, as for the tocotrienol derivatives, carboxylic acid esters of tocotrienol are preferably used.

Examples of vitamin A types include letinol, 3-hydroletinol, letinal, 3-hydroletinal, letinoic acid, 3-dehydroletinoic acid and vitamin A acetate. Examples of the vitamin D types include vitamin D types ranging from vitamin $D_2$ to vitamin $D_7$ or the like. Examples of the other fat-soluble vitamin substances include esters such as vitamin E nicotinate; and vitamin K types ranging from vitamin $K_1$ to vitamin $K_3$ or the like.

Further, examples of the fat-soluble vitamin types also include erythorbic acid fatty acid esters such as erythorbic acid palmitic acid eater and erythorbic acid tetraisopalmitic acid eater; and fatty acid esters of vitamin $B_6$ such as pyridoxine dipalmitic acid, pyridoxine tripalmitic acid, pyridoxine dilauric acid and pyridoxine dioctanoic acid.

As for the other oils and fats, a liquid grease (fatty oil) and a solid grease (fat) at ordinary temperature are exemplified.

Examples of the liquid grease include olive oil, camellia oil, macadenia nut oil, castol oil, avocado oil, evening primrose oil, turtle oil, corn oil, mink oil, rapeseed oil, egg-yolk oil, sesame oil, persic oil, wheat germ oil, sasanqua oil, linseed oil, safflower oil, cottonseed oil, perilla oil, soybean oil, peanut oil, tea seed oil, kaya oil, rice bran oil, China wood oil (tung oil), Japan wood oil (tung oil), Jojoba oil, germ oil, triglyceride, glyceryl trioctanoate, glyceryl tripalmitate, salad oil, safflower oil, palm oil, coconut oil, peanut oil, almond oil, hazelnut oil, walnut oil, grape seed oil, scqualene and scqualane.

Examples of the solid grease include beef tallow, hydrogenated beef tallow, neats-foot oil, beef bone fat, mink oil, egg-yolk oil, pig fat, horse oil, sheep oil, hydrogenated oil, cacao oil, palm oil, hydrogenated palm oil, Japan wax, Japan wax-karnel oil and hydrogenated castor oil.

Among the above-described oils and fats, a coconut oil that is a medium-chain triglyceride is preferably used from the viewpoints of both particle size and stability of the emulsion stability.

In order to improve properties in the composition, it is preferred for the oil component in the present invention to contain a compound selected from the group consisting of tocopherol, tocotrienol and derivatives thereof that are included in the fat-soluble vitamin (hereinafter, the compound is referred to as "tocopherols", when appropriate) together with other oil phase component (s).

In the case where the above-described tocopherols are used in combination in the carotenoid-containing oil phase composition, they may be used in combination in a range of preferably from 5% by mass to 35% by mass and more preferably from 7% by mass to 20% by mass with respect to a total mass of the oil component.

In the present invention, the content of the carotenoid-containing oil phase composition in the carotenoid-containing composition varies depending on the form of the carotenoid-containing composition to be targeted. However, from the viewpoint of exerting functions of the oil components, the content in the case of emulsion composition is preferably from 0.1% by mass to 50% by mass, more preferably from 0.5% by mass to 25% by mass and still more preferably from 0.2% by mass to 10% by mass in terms of the total amount of the oil phase components. Meanwhile, the content in the case of powder composition is preferably from 10% by mass to 50% by mass, more preferably from 10% by mass to 40% by mass and still more preferably from 10% by mass to 30% by mass in terms of the total mass of the components.

In addition to the above-described components, an emulsifying agent that can be used as an oil phase component may be contained. Examples of the emulsifying agent that can be used as the oil phase component include an emulsifying agent having HLB of 7 or less among the emulsifying agent described below.

[Aqueous composition]

In the production process of the oil-in-water type emulsion in the production method of the present invention, an aqueous composition containing a water-soluble emulsifying agent and the above-described carotenoid-containing oil phase composition are emulsified under pressure to obtain an oil-in-water type emulsion composition.

The aqueous composition that can be used to obtain the carotenoid-containing composition of the present invention is composed of an aqueous medium, especially water, and contains at least an emulsifying agent.

The emulsifying agent may be any of anionic surfactants, cationic surfactants, amphoteric surfactants and nonionic surfactants.

Further, from the viewpoint of emulsifying capacity, HLB of the emulsifying agent is preferably 10 or more, and more preferably 12 or more. When HLB is too low, sometimes the emulsifying capacity may become insufficient. In addition, from the viewpoint of form-inhibiting effect, an emulsifying agent having HLB of from 5 to less than 10 may be used in combination.

Herein, the term HLB indicates a hydrophile-lipophile balance, and ordinarily used calculation formula, for example, Kawakami's formula or the like can be used The Kawakami's formula is shown below.

$$HLB=7+11.7 \log (M_w/M_o)$$

Herein, $M_w$ represents a molecular weight of a hydrophilic group(s), and $M_o$ represents a molecular weight of a hydrophobic group(s).

Further, numerical values of HLB described in catalogues and the like may be used.

Further, as can be understood from the above formula, emulsifying agents having desired HLB values can be obtained by utilizing the additive property of HLB.

The content of the emulsifying agent in the carotenoid-containing composition generally varies depending on the form of the composition. However, in the case of an emulsion composition, the content is preferably from 0.5% by mass to 30% by mass, more preferably from 1% by mass to 20% by mass and still more preferably from 2% by mass to 15% by mass with respect to a total mass of the components. Meanwhile, in the case of a powder composition, the content is preferably from 0.1% by mass to 50% by mass, more preferably from 5% by mass to 45% by mass and still more preferably from 10% by mass to 30% by mass with respect to a total mass of the components. The above-described range is preferable in the points that if the content is within the range, interfacial tension between oil phase/poor solvent are easily reduced and excessive amounts of the emulsifying agent are not added, so that it hardly causes a problem such as occurrence of serious foaming in the dispersion composition.

Further, the total mass of the emulsifying agent, in either of the powder composition or the emulsion composition, may be used in a range of from 0.1 to 10 times with respect to a total mass of the oil components containing carotenoid. From the viewpoints of refinement of dispersion particles and foaming inhibition, a range of from 0.5 to 8 times is preferable, and a range of from 0.8 to 5 times is especially preferable. These ranges make it possible to provide good dispersion stability of the composition.

Among the emulsifying agents, nonionic surfactants are preferred from the points of low-irritating property and low-environmental impact. Examples of the nonionic surfactants include sucrose fatty acid ester, polyglyceryl fatty acid ester, organic acid monoglyceride, propylene glycol fatty acid ester, polyglyceryl condensed ricinoleate, sorbitan fatty acid ester, and polyoxyethylene sorbitan fatty acid ester.

It is preferred from the viewpoint of stability of dispersion particles in the composition that the sucrose fatty acid esters are those in which the carbon number of a fatty acid that constitutes the sucrose fatty acid ester is from 12 to 20, and more preferably from 14 to 16.

Preferable examples of the sucrose fatty acid ester include sucrose dioleate, sucrose distearate, sucrose dipalmitate, sucrose dimyristic acid ester, sucrose dilaurate ester, sucrose monooleate, sucrose monostearate, sucrose monopalmitate, sucrose monomyristic acid ester and sucrose monolaurate ester. Among these, sucrose monooleate, sucrose monostearate, sucrose monopalmitate, sucrose monomyristic acid ester and sucrose monolaurate ester are preferred.

In the present invention, these sucrose fatty acid esters may be used singly or by mixture thereof.

The polyglyceryl fatty acid ester contained in an aqueous-phase composition is an ester of polyglycerin having average degree of polymerization of 2 or more, preferably from 6 to 15 and more preferably from 8 to 10, and a fatty acid having 8 to 18 carbon atoms, such as caplylic acid, capric acid, lauric acid, myristic acid, palmitic acid, stearic acid, oleic acid, and lynoleic acid.

Preferable examples of the polyglyceryl fatty acid ester include hexaglyceryl monooleate, hexaglyceryl monostearate, hexaglyceryl monopalmitate, hexaglyceryl monomyristate, hexaglyceryl monolaurate, decaglyceryl monooleate, decaglyceryl monostearate, decaglyceryl monopalmitate, decaglyceryl monomyristate and decaglyceryl monolaurate.

Among these, more preferred are decaglyceryl monooleate (HLB=12), decaglyceryl monostearate (HLB=12), decaglyceryl monopalmitate (HLB=13), decaglyceryl monomyristate (HLB=14) and decaglyceryl monolaurate (HLB=16).

These polyglyceryl fatty acid esters may be used singly or by mixture thereof.

The sorbitan fatty acid esters in the present invention are preferably those in which the carbon number of a fatty acid is 8 or more, and more preferably from 12 or more. Preferable examples of the sorbitan fatty acid ester include sorbitan monocaplylate, sorbitan monolaurate, sorbitan monostearate, sorbitan sesquistearate, sorbitan tristearate, sorbitan isostearate, sorbitan sesquiisostearate, sorbitan oleate, sorbitan sesquioleate and sorbitan trioleate,

In the present invention, these sorbitan fatty acid esters may be used singly or by mixture thereof.

The polyoxyethylene sorbitan fatty acid esters are preferably those in which the carbon number of a fatty acid is 8 or more, and more preferably from 12 or more. Further, the ethyleneoxide length (addition mole number) of the polyoxyethylene is preferably from 2 to 100 and more preferably from 4 to 50.

Preferable examples of the polyoxyethylene sorbitan fatty acid ester include polyoxyethylene sorbitan monocaplylate, polyoxyethylene sorbitan monolaurate, polyoxyethylene sorbitan monostearate, polyoxyethylene sorbitan sesquistearate, polyoxyethylene sorbitan tristearate, polyoxyethylene sorbitan isostearate, polyoxyethylene sorbitan sesquiisostearate, polyoxyethylene sorbitan oleate, polyoxyethylene sorbitan sesquioleate and polyoxyethylene sorbitan trioleate.

These polyoxyethylene sorbitan fatty acid esters may be used singly or by mixture thereof.

Further, phospholipids such as lecithin may be contained as the emulsifying agent in the present invention.

The phospholipid that can be used in the present invention contains a glycerin skeleton, a fatty acid residue and a phosphoric acid residue as essential constituents, further a base, a polyalcohol or the like being bound thereto. The phospholipid may be referred to as a lecithin. The phospholipid has a hydrophilic group and a hydrophobic group in the molecule, and therefore has been widely used as an emulsifier in the field of foods, medical goods and cosmetics from the past.

Industrially, a substance in which purity of lecithin is 60% or more is used as lecithin and also can be used in the present invention. However, from the viewpoint of formation of fine droplet particle size and stability of functional oil components, a material that is generally called high-purity lecithin is preferable. This material has lecithin purity of 80% or more and more preferably lecithin purity of 90% or more.

Examples of the phospholipid include various kinds of hitherto-known substances obtained by extraction and separation from living bodies of plants, animals and microorganism.

Specific examples of the above-described phospholipid include various kinds of lecithin derived from plants such as soybean, corn, peanut, rapeseed and wheat; animals such as egg yolk and cow; and microorganism such as coli bacterium.

Examples of lecithin identified by compound names include glycerolecithin such as phosphatidic acid, phosphatidyl glycerin, phosphatidyl inositol, phosphatidyl ethanol amine, phosphatidyl methylethanol amine, phosphatidyl choline, phosphatidyl serine, bisphosphatidic acid, diphosphatidyl glycerin (cardiolipin) and sphingolecithin such as sphingomyelin.

Further in the present invention, besides the above-described high-purity lecithin, hydrogenated lecithin, enzyme degradation lecithin, enzyme degradation hydrogenated lecithin, hydroxy-lecithin or the like may be used. The above-described lecithin that can be used in the present invention may be used singly or in a form of mixture of two or more kinds.

The carotenoid-containing composition of the present invention preferably contains a water-soluble encapsulating agent in order to protect oil droplets in a powderization process or during storage of powder in the case where the carotenoid-containing composition is formed as a powder composition. This can keep the particle size of oil droplets in a microscopic state, and also can reduce deterioration of carotenoid components in the oil droplets.

Further, the water-soluble encapsulating agent, when the powder composition is re-dissolved in water, can provide good water-dispersibility of the oil droplets, and also can provide good transparency after re-dissolution.

The water-soluble encapsulating agent is preferably at least one polysaccharide selected from fructose polymer and oligomer composed of sugar units containing at least two fructose units (hereinafter, referred to simply as "fructose polymer or oligomer").

The fructose polymer or oligomer in the present invention refers to a polymer or oligomer which contains fructose as a repeating unit, and also which is composed of sugar units in which multiple sugar units are combined together by dehydration condensation. In present invention, the polysaccharide in which the number of the sugar repeating units containing fructose units is less than 20 is referred to as a fructose oligomer. Meanwhile, the polysaccharide in which the number of the sugar repeating units containing fructose units is 20 or more is referred to as a fructose polymer.

The number of the sugar repeating units is preferably from 2 to 60 and more preferably from 4 to 20, from the viewpoints of dry suitability and miniaturization of oil droplets at a time of re-dissolution. When the number of the repeating unit (polymerization degree of fructose) is 2 or more, hydroscopic property is not too strong and a problem of adhesion to a drying container during drying and resultant reduction in collection rate can be effectively prevented. Meanwhile, when the number of the repeating unit is 60 or less, coarsening of oil droplets at the time of re-dissolution in water can be effectively prevented.

The fructose polymer or oligomer may contain, besides fructose, other monosaccharide (s) at the end or in a chain of the molecule. Examples of the other monosaccharide unit that can be contained in this case include glucose (glucose sugar), galactose, mannose, idose, altrose, gulose, talose, allose, xylose, arabinose, lyxose, ribose, threose, erythrose, erythrulose, xylulose, ribulose, psicose, sorbose, and tagatose. However, the other monosaccharide unit is not limited to these. Among these monosaccharides, glucose is preferable from the viewpoint of availability. As for the binding position, presence of the monosaccharide at the end of fructose chain is preferable from the viewpoint of miniaturization of oil droplets at the time of re-dissolution.

In the case where a sugar group other than fructose is contained, its content ratio is 50% or less and preferably 30% or less in terms of polymerization degree with respect to the unit number of fructose from the viewpoints of dry suitability and miniaturization of oil droplets at the time of re-dissolution.

As for the water-soluble encapsulating agent that can be preferably used in the present invention from the viewpoints of storage stability of the colorant and availability, inulin is exemplified. Inulin in the present invention refers to fructose polymer or oligomer having a (one) glucose at the end of the molecule. Inulin is known as being present widely in nature and a high level of Inulin is contained in chicory, sun choke, dahlia, garlic, Chinese chive, onion and the like. Details of inulin are described in Hand book of Hydrocolloides, G. O. Phillips, P. A. Williams Ed., 397-403, (2000) CRC Press. Generally, the chain length is represented by expressing the glucose unit as G and the fructose unit as F. In the inulin in the present invention, sucrose expressed by GF is not included.

Inulin that is ordinarily extracted from natural substances is a polymer or oligomer ranging from GF2 (kestose), GF3 (nystose), GF4 (fructosylnystose) to GF60 or a similar extent, and their mixture.

In the present invention, inulin may be commercially-available products that are obtained by a process of separating and extracting by hot water from roots of chicory, sun choke, dahlia or the like, and then concentrating the water extract, and then powderizing it by spray-drying. Examples of the commercially-available products may include FRUTAFIT (produced by SENSUS) extracted from a root of chicory, BENEO (ORAFTI) extracted from a root of chicory, a reagent derived from dahlia (Wako Pure Chemical Industries, Ltd., and Sigma Ltd.) and a reagent extracted from a root of chicory Sigma Ltd.).

Further, the fructose polymer or oligomer in the present invention may include a product that is produced from sucrose using dislocation activity of fructan in β-fructofuranosidase. Examples of this type include FUJI FF (Fuji nihon seito Corporation) and GF2 (Meiji Seika Pharma, Company, Limited).

Inulin used in the present invention is preferably from 2 to 60 in terms of the number of repeating unit of fructose (degree of polymerization) from the viewpoint of miniaturization of oil droplets at the time of re-dissolution, and the polymerization degree of fructose is more preferably from 4 to 20 from the viewpoints of adhesion property to an apparatus at the time of spray-drying and solubility in water.

It is preferable that the fructose polymer or oligomer in the present invention already be added at the time of emulsification. However, a partial or all of the fructose polymer or oligomer may be added after emulsification.

Further, other water-soluble polymer or oligomer may be used in combination with the fructose polymer or oligomer. Examples of the other water-soluble polymer or oligomer include agarose, starch, carrageenan, gelatin, xanthane gum, gellan gum, galactomannan, casein, tragacanth gum, xylogulucan, β-glucan, curdlan, water-soluble soy fiber, chitosan, alginic acid and sodium alginate. However, the other water-soluble polymer or oligomer is not limited to these.

The water-soluble encapsulating agent in the carotenoid-containing composition is preferably included in an amount from 0.5 to 50 times the total mass of oil components in the composition, more preferably from 1 to 20 times, still more preferably from 1 to 10 times, and still more preferably from 2 to 5 times, from the viewpoints of retention of shape and solubility.

Further, the water-soluble encapsulating agent may be contained in the aqueous phase of the carotenoid-containing composition. The water-soluble encapsulating agent may be contained in the aqueous phase composition at the time of emulsification under pressure as described below, or the water-soluble encapsulating agent may be added to the aqueous phase of the carotenoid-containing composition after emulsification under pressure.

[Other components]

Besides the above-described components, components that are ordinarily used in the field of foods, cosmetics and the like may be appropriately compounded in the carotenoid-containing composition of the present invention in accordance with the configuration of the composition. The addition components may be compounded as a component of the oil phase component mixed liquid, the carotenoid-containing composition, or the aqueous phase composition in accordance with properties of the addition components, or may be compounded as a component of addition to the aqueous phase of the carotenoid-containing composition.

Examples of the above-described other components include: polyalcohols such as glycerin and 1,3-butyleneglycol; monosaccharides or polysaccharides such as glucose, fructose, lactose, maltose, sucrose, pectin, κ-carrageenen, locust bean gum, guar gum, hydroxypropyl guar gum, xanthane gum, karaya gum, tamarind seed polysaccharide, gum Arabic, tragacanth gum, hyaluronic acid, sodium hyaluronate, sodium chondroitin sulfate and dextrin; sugar alcohols such as sorbitol, mannitol, multitol, lactose, maltotriitol and xylitol; inorganic salts such as sodium chloride and sodium sulfate; protein having molecular weight of more than 5000 such as casein, albumin, methylated collagen, hydrolyzed collagen, water-soluble collagen and gelatin; synthetic polymers such as carboxyvinyl polymers, sodium polyacrylate, polyvinyl alcohol, polyethylene glycol and ethyleneoxide-propyleneoxide block copolymer, water-soluble cellulose derivatives hydroxyethylcellulose•methylcellulose; flavonoids (catechin, anthocyanine, flavone, isoflavone, flavane, flavanone, rutin), phenolic acids (chlorogenic acid, ellagic acid, gallic acid, propyl gallate), lignans, curucumins and coumarins. The other components may be contained in accordance with their functions, for example, as a functional component, an excipient, a viscosity modifier, a radical scavenger and the like.

In addition, other additives that are usually used for the intended use, for example, various kinds of medically effective components such as a pH adjuster, a pH buffer, a ultraviolet-ray absorber, perfume and a colorant, may be used in combination.

[Production method]

In the production method of a carotenoid-containing composition according to the present invention, a carotenoid-containing oil phase composition that has been obtained in the process of preparing the carotenoid-containing oil phase composition, and an aqueous phase composition are emulsified under pressure in the process of preparing an oil-in-water type emulsified composition, thereby obtaining the carotenoid-containing composition as the oil-in-water type emulsified composition.

By this method, a carotenoid-containing composition is obtained which is an oil-in-water type emulsion in which oil droplets (dispersion particles) containing oil components including a crystalline carotenoid are finely dispersed in water, and crystallization of carotenoid has been suppressed.

Although the ratio (mass) of an oil phase and an aqueous phase in the emulsification is not particularly limited, the ratio (% by mass) of oil phase/aqueous phase is preferably from 0.1/99.9 to 50/50, more preferably from 0.5/99.5 to 30/70, and still more preferably from 1/99 to 20/80.

By setting the ratio of oil phase/aqueous phase to 0.1/99.9 or more, reduction in active components is prevented, which results in a favorable trend that a practical problem of the emulsion composition is not caused. Further, by setting the ratio of oil phase/aqueous phase to 50/50 or less, reduction in concentration of the emulsifier is prevented, which results in a favorable trend that emulsion stability of the emulsified composition is not deteriorated.

The emulsification under pressure may be performed by one step-operation of the emulsification. However, it is preferable to perform two or more step-operations of the emulsification, from the viewpoint of obtaining uniform and microscopic emulsified particles.

Specifically, it is especially preferable to use a combination of two or more kinds of emulsification devices in a manner such that emulsification is performed by way of a high-pressure homogenizer or the like in addition to the one step-operation of the emulsification in which emulsification is performed using an ordinary emulsification device (for example, stirrer, impeller agitation, homo-mixer, continuous-flow type shearing machine) using a shearing force. By using a high-pressure homogenizer, the emulsion can be aligned with uniform and fine particles of oil droplets. Further, a plurality of operations may be additionally performed in order to make the particle size of oil droplets more uniform.

As an emulsification means that can be used herein, use can be made of any of generally known emulsification techniques such as a spontaneous emulsion method, an interfacial chemical emulsion method, an electric emulsion method, a capillary emulsion method, a mechanical emulsion method, a supersonic emulsion method and the like.

As a useful method for making the emulsion particles more microscopic, an interfacial chemical emulsion method such as a PIT emulsion method, a gel emulsion method and the like is known. This method has an advantage in that consumption energy is low, and therefore the method is suitable in a case of finely emulsifying a material that is easy to deteriorate by heat.

Further, as a generally-used emulsion method, a method of using a mechanical force is suitably used, that is, the method of tearing apart oil droplets by applying a shearing force thereto from the outside. The most-general force of the mechanical force is a high-speed and high-shearing mixer. As such mixer, mixers that are called a homo-mixer, a disper mixer or a super-mixer are offered commercially.

As another mechanical emulsification device that is useful for particle-size reduction, a high-pressure homogenizer is available and various kinds of devices are offered commercially. The high-pressure homogenizer is capable of applying greater shearing force than a stirring method, and accordingly even the amount of an emulsifier is relatively small, particle- size reduction can be realized.

There are two main types of high-pressure homogenizer: one is a chamber type high-pressure homogenizer having a fixed throttling section and a homogeneous valve type high-pressure homogenizer in which the divergence of throttle is governed.

Examples of the chamber type high-pressure homogenizer include MICROFLUIDIZER (manufactured by Microfluidics Corporation), NANOMIZER (manufactured by Yoshida Kikai Co., Ltd.) and ALTIMIZER (manufactured by Sugino Mashine Limited).

Examples of the homogeneous valve type high-pressure homogenizer include Gaulin-type homogenizer (manufactured by APV Company), Rannie-type homogenizer (manufactured by Rannie Company), HIGH-PRESSURE HOMOGENIZER (manufactured by Nio Soabi Company), HOMOGENIZER (manufactured by SANNWA MACHINERY TRADING CO., LTD.), HIGH-PRESSURE HOMOGENIZER (manufactured by IZUMI FOOD MACHINERY CO., LTD.), and ULTRAHIGH-PRESSURE HOMOGENIZER (manufactured by IKA Corporation).

There is a ultrasonic homogenizer as a dispersing device having a relatively good energy efficiency and a emulsification device having a simple structure. Examples of a high-power ultrasonic homogenizer that can be produced include ultrasonic homogenizer US-600, ibid. US-1200T, ibid. RUS-1200T and ibid. MUS-1200T (all manufactured by NISSEI Corporation), ultrasonic processor UIP 2000, ibid. UIP 4000, ibid. UIP 8000 and ibid. UIP 1600 (all manufactured by Heilscher). These high-power ultrasonic homogenizers are used at frequency of 25 kHz or less, and preferably at frequency of from 15 to 20 kHz.

Further, as another known emulsifying means, a method of using a device that does not include an extraneous agitation section and needs only low energy is also useful. Examples of the device include a static mixer, a micro channel, a macro mixer and a membrane emulsification device.

The temperature condition at the time of emulsion dispersion in the present invention is not particularly limited. However, from the viewpoint of stability of functional oil components, the range of from 10 to 100°C is preferable and a favorable range can be appropriately selected depending on a melting point of the functional oil component to be handled.

Further, in a case of using a high-pressure homogenizer in the present invention, the pressure is preferably 50 M Pa or more, more preferably from 50 M Pa to 280 M Pa, and still more preferably from 100 M Pa to 280 M Pa and the processing is preferably performed at this pressure.

Further, from the viewpoint of keeping particle size of the dispersion particles, it is preferred that an emulsified liquid that is an emulsion dispersed composition is cooled through some sort of cooling machine within 30 seconds, preferable within 3 seconds immediately after the emulsified liquid has passed through a chamber.

The production method of the present invention may include drying the oil-in-water type emulsified dispersion obtained by a process of preparing an oil-in-water type emulsified dispersion to obtain a powder composition (hereinafter, may be referred to as "a powderization process"). This method makes it possible to obtain a carotenoid-containing composition as a powder composition. The carotenoid-containing composition as a powder composition is a composition that has storage stability due to the powderization form, and in addition, a composition in which crystallization of crystalline carotenoid has been suppressed in the powder composition as well as the emulsified composition in which the powder composition has been re-dissolved in an aqueous medium.

As for the drying means used in the powderization process, known drying means may be used. Examples of the drying means include natural drying, heat drying, hot air drying, high-frequency drying, ultrasonic drying, reduced-pressure drying, vacuum drying, freeze dry and spray drying. These means may be used singly, or in combination of two or more kinds thereof.

In the present invention, reduced-pressure drying, vacuum drying, freeze dry and spray drying are preferable, because functional materials that are weak against heat are often contained. Further, as one of the vacuum drying methods, a method of conducting vacuum (reduced-pressure) drying while keeping the temperature in a range of 0°C or lower and freezing temperature or higher is also preferred.

In a case of vacuum drying or reduced-pressure drying, the drying is preferably conducted by repeating concentration while gradually increasing the degree of reduced pressure in order to avoid scatter of the liquid due to bumping.

In the present invention, the freeze dry in which ice is sublimated from a material in a frozen state to remove water is preferred. The freeze dry method has a great advantage such that since the dry process is usually performed at 0°C or lower, ordinarily at a range of from about -20°C to about -50°C, heat denaturation of the material is not caused, and in the course of water recovery, taste, color, nutritional value, shape, texture and the like are easy to restore to their state before drying.

Examples of the commercially available freeze dryer include FREEZE DRYER VD-800F (TAITEC Corporation), FLEXI-DRY MP (FTS SYSTEMS INC.), DURATOP·DURASTOP (FTS SYSTEMS INC.), TAKARA FREEZE-DRYER Model A (TAKARAATM), DESKTOP FREEZE-DRYER FD-1000 (TOKYO RIKAKIKAI CO., LTD.), VACCUM FREEZE-DRYER FD-550 (TOKYO RIKAKIKAI CO., LTD.) and VACCUM FREEZE-DRYER (TAKARA SEISAKUSYO). However, the present invention is not limited thereto.

Further, in the present invention, a spray-drying method is especially preferred as a drying means from the viewpoint of a good valance between production efficiency and quality. The spray dry is a sort of convective-hot air drying. The liquid composition is sprayed as fine particles of several 100 $\mu$m or less in a hot air and resultantly drops in a tower while being dried, whereby the composition is collected as a solid powder thereof. Though the material is temporarily exposed to hot air, increase of temperature doe not become too high because of very short exposure time and vapor latent heat, and therefore heat denaturation of the material is not easy to be caused and a change due to water recovery is small as is the case with freeze dry. In a case of a material that is very weak against heat, it is also possible to feed cold air in stead of hot air. In this case, relatively milder drying can be favorably realized, though the drying performance is reduced.

Examples of the commercially available spray dryer include a spray dryer SPRAY DRYER SD-1000 (TOKYO RIKAKIKAI CO., LTD.), SPRAY DRYER-8i (OHKAWARA KAKOHKI Co., LTD.), CLOSED SPRAY DRYER CL-12 (OHKAWARA KAKOHKI Co., LTD.), SPRAY DRYER ADL 310 (YAMATO SCIENTIFIC CO., LTD.), MINISPRAY DRYER B-290 (BUCHI), PJ-MiniMax (Powdering Japan) and PHARMASD (GEANiro). However, the present invention is not limited thereto.

Further, it is also preferred to produce granular particles exerting excellent handling ability at the same time as drying by using a device by which both drying and granulation are performed at the same time, such as fluid-bed granulation dryer (POWREX CORPORATION) and spray dryer with a built-in fluid-bed FSD (GEA Niro).

The powder composition obtained by the production method of the present invention has a water recovery property, that is, a property of restoring the condition of the oil-in-water type emulsified composition before drying, when the powder composition is re-dissolved (re-dispersed) again in water.

The carotenoid-containing composition obtained by the production method of the present invention is an oil-in-water type emulsified composition or a powder composition obtained by powderization of the same.

The average particle diameter in the carotenoid-containing composition means a particle diameter of the dispersion particles (oil droplets) in the emulsified composition in a case of the oil-in-water type composition, and means a particle size of the dispersion particles (oil droplets) in a 1 % by mass aqueous solution (at the time of re-dissolution) in a case of the powder composition.

The particle size of the dispersion particles can be measured using a commercially-available particle size distribution measuring device or the like. As for the particle size distribution measuring method, optical microscopy, laser confocal microscopy, electron microscopy, atomic force microscopy, static light-scattering method, laser diffractometry, dynamic light-scattering method, centrifugal precipitation method, electric pulse measuring technique, chromatographic method and attenuation method of ultrasonic wave are known. Devices in accordance with their principles are commercially available.

The dynamic light-scattering method is preferable in the particle size measurement of the dispersion particles from the viewpoints of particle size range and easiness of measurement in the present invention. Examples of the commercially-available measuring device using the dynamic light-scattering method include NANOTRAK UPA (Nikkiso Co., Ltd.), dynamic light-scattering method particle size distribution measuring device LB-550 (HORIBA, Ltd.) and concentrated system particle size analyzer FPAR-1000 (Otsuka Electronics Co., Ltd.). However, as the particle size in the present invention, a value obtained by measurement at 25°C using the particle size analyzer FPAR-1000 (Otsuka Electronics Co., Ltd.) is adapted.

That is, in the measuring method of the particle size, the particle size in terms of median size (d=50) is measured using the particle size analyzer FPAR-1000 (Otsuka Electronics Co., Ltd.) in a manner such that in a case of the oil-in-water type composition, it is diluted 20-fold with pure water, meanwhile in a powder composition, it is diluted with pure water so that the solid content becomes 1 % by mass.

Further, the particle size of the dispersion particles may be adjusted by factors such as agitation conditions in the production method (shearing force · temperature · pressure) and ratio of oil phase and aqueous phase, besides the components of the composition.

The particle size of the carotenoid-containing composition in the present invention is preferably from 50 nm to 300 nm from the viewpoints of transparency and absorbing property, and more preferably from 50 nm to 200 nm and most preferably from 50 nm to 150 nm from the viewpoint of transparency.

Further, according to the present invention, there can be obtained a carotenoid-containing composition that contains at least lycopene, at least 90% of the lycopene being present in the non-crystalline state in the composition. The carotenoid component contained in the carotenoid-containing composition is favorable, as long as at least 90 % by mass to 100 % by mass of lycopene is non-crystalline and more favorable from the viewpoint of dynamic absorption property in the case where from 95 % by mass to 100 % by mass of the lycopene is non-crystalline.

In order to confirm that the lycopene is present in the non-crystalline state, any of known means for detecting a crystal structure may be used. The target can be determined as being non-crystalline on the ground that detection of crystalline body cannot be confirmed by any of these known means. Especially in the present invention, confirmation of non-crystallinity is preferably conducted on the basis of the presence of DSC endothermic peak. Specifically, both endothermic temperature and exothermic temperature were measured by one cycle of temperature rising-temperature decrease (15°C/min) within a temperature range of from 30°C to 200°C using DSC Q 2000 (TA Instruments Japan) in a manner such that the emulsified composition is freeze-dried to be dewatered meanwhile the powder composition is in the power state. The target is determined as being non-crystalline on the ground that the presence of perceptible endothermic peak is not recognized.

For example, by comparing an endothermic amount of the endothermic peak originated from a lycopene crystal in the carotenoid component with the endothermic amount of the endothermic peak originated from the lycopene crystal sample measured using differential scanning calorimetric measurement (Differential scanning calorimetry, DSC), it can be confirmed that at least 90 % by mass of the lycopene contained in the carotenoid component is non-crystalline. In order to conform that the lycopene is non-crystalline in the present invention, this DSC-using method is adopted.

In addition, by comparing a spectrum of a carotenoid-containing composition of the present invention obtained from X-ray diffraction with the spectrum of the carotenoid crystal sample, it can be also confirmed that the lycopene is non-crystalline.

Further, the content ratio of the lycopene that is non-crystalline can be converted from the results obtained by DSC peak area or XRD (X-ray diffraction) in a manner such that use is made of a commercially-available carotenoid reagent that is a crystal body, provided that the content ratio of the carotenoid reagent is 100%. Examples of the commercially-available carotenoid reagent that is a crystal body include a biochemical reagent available from Wako Pure Chemical Industries, Ltd.

The carotenoid-containing composition of the present invention is a carotenoid-containing composition such that crystallization of the lycopene is suppressed whereby a desirable effect due to the carotenoid can be fully expected. Accordingly, the carotenoid-containing composition can be favorably applied to a food composition, a cosmetic composition and a pharmaceutical composition.

Further, to foods or cosmetics containing the emulsion composition of the present invention, if needed, any of components that can be added to the foods or the cosmetics may be added appropriately. Especially in the case where the component is used for foods, a powdery food with a long shelf life can be provided, and when dissolved in an aqueous medium, the powdery food becomes a dispersed composition that has fine dispersion particles and excellence in transparence.

Foods, cosmetics or the like containing the carotenoid-containing composition of the present invention may exhibit an effect that may not be exerted due to the presence of crystal body, for example, good absorbing property of carotenoid.

The cosmetics composition is favorably used in, for example, lotion, beauty essence, milky lotion, massage mask, facial mask, shampoo cosmetics, fragrance cosmetics, liquid body cleaning preparations, UV care cosmetics, deodorant cosmetics, cosmetics for oral health and the like.

The foods is favorably used in, not only common foods such as a nutrition-supplement drink, a revitalizer, a paratable drink and a frozen dessert, but also tablet-shape · granule-shape · capsule-shape dietary supplements.

In a case of use for functional foods, although an addition amount of the powder composition according to the present invention cannot be simply generalized because it varies depending on the kind and the intended use of a product, the powder composition can be used by adding it so as to become in a range of from 0.01 to 10% by mass and preferably from 0.05 to 5% by mass, with respect to the product. If the addition amount is 0.01% by mass or greater, exertion of a desired effect may be prospective and meanwhile if it is 10% by mass or lower, appropriate effects may be often exerted efficiently.

EXAMPLES

Hereinafter, the present invention is described in reference to examples. However, the present invention is not limited thereto. Note that the numerical numbers expressed by "part" and "%" respectively are based on mass standard, unless it is explicitly stated otherwise.

[Example 1]

<Preparation of oil phase composition>

The oil phase components (excluding mixed tocopherol) described below were adjusted so as to become a range of 160°C to 165°C from room temperature, and were stirred and dissolved while heating for 20 minutes to obtain a carotenoid-containing oil phase composition. The obtained carotenoid-containing oil phase composition was adjusted at 60°C to keep it hot, and mixed tocopherol was added while stirring to obtain oil phase composition 1.

< Preparation of aqueous phase composition >

The aqueous phase components described below were dissolved by mixture and agitation while heating at 70°C. Then, a coarse dispersion was performed using 600 W ultrasonic homogenizer US-150T, manufactured by NISSEI Corporation) to obtain aqueous phase composition 1.

[Oil phase composition 1]

- Lycopene paste (lycopene concentration: 18%) 8.9g
- Diglyceryl monostearate 0.9g
- Calcium ascorbate (50% solution) 7.1g
- Mixed tocopherol 1.3g

[Aqueous phase composition 1]

- Sucrose ester of lauric acid 11.1g
- Lecithin 1.8g
- Inulin 25.6g
- Water 246.9g

Note that LYCOPENE 18 (Kyowa Wellness Co., Ltd.) as the lycopene paste, NIKKOL DGMS (HLB=5.0, manufactured by NIKKO CHEMICALS CO., LTD.) as diglyceryl monostearate, and RIKEN E OIL 800 (manufactured by RIKEN VITAMIN CO., LTD.) were used respectively. Further, RYOTO Sugar Ester L-1695 (HLB=16, manufactured by Mitsubishi Kagaku Foods Corporation) as sucrose lauric acid ester, LECION P (manufactured by RIKEN VITAMIN CO., LTD.) as lecichin, and FUJI FF (manufactured by Fuji nihon seito Corporation) as inulin were used respectively. Further, the melting point of LYCOPENE 18 was 153°C.

<Preparation of emulsion>

Oil phase composition 1 was kept at 60°C while stirring. To this composition, the above-prepared aqueous phase composition 1 kept at 70°C was added and subjected to emulsification for 3 minutes using 600 W ultrasonic homogenizer, thereby obtaining coarsely dispersed emulsion 1 (lycopene concentration: 0.53%).
Next, the coarsely dispersed emulsion 1 was subjected to high-pressure emulsification processing under the conditions of pressure: 245 M Pa and 30°C using STIRBURST MINI (manufactured by Sugino Machine Limited) in a manner such that this process was repeated four times. By this process, emulsion 1 was obtained.
Next, spray dry of the obtained emulsion 1 was performed under the conditions of spray pressure: 0.15 M Pa and exit temperature: 80°C, throughput: 7 ml/minute, using spray dry (SPRAY DRYER Model DL310, manufactured by YAMATO SCIENTIFIC CO., LTD.). The resultant powder was collected by cyclone to obtain powder composition 1 with 3% of lycopene concentration.

[Examples 2 and 5, Reference Examples 3 and 4, and Comparative Examples 1 to 2]

Oil phase compositions 2 to 5, 7 and 8 and aqueous phase compositions 2 to 5, 7 and 8 were obtained in the same manner as Example 1, except that the kind and content of the oil phase components and the aqueous phase components were changed as shown in Table 1. Emulsification was performed using the oil phase compositions 2 to 5, 7 and 8 and the aqueous phase compositions 2 to 5, 7 and 8 in the same manner as Example 1, thereby obtaining emulsions 2 to 5, 7 and 8. Further, spray dry was performed to obtain powder compositions 2 to 5, 7 and 8.
Note that in Table 1, COCONAD MT (HLB=1, manufactured by Kao Corporation) as tri(caprylic acid/capric acid)glyceryl, HEXAGLYN 5-SV (glycerin number: 7, stearic acid number: 5, manufactured by Nikko Chemicals Co., Ltd.) as hexaglyceryl pentastearate, ASCOBIC ACID PM (manufactured by SHOWA DENKO K.K.) as ascorbic acid-phosphate magnesium salt and DECAGLYN 1-SV (glycerin number: 10, stearic acid number: 1, HLB=12.0, manufactured by Nikko Chemicals Co., Ltd.) as decaglyceryl monostearate were used respectively.

[Ref. Example 6]

Emulsion 6 was obtained by preparation of each phase composition and emulsification in the same manner as Example 1, except that the kind and content of the oil phase components and the aqueous phase components were changed as shown in Table 1 and spray dry was not performed. Reference Example 6 is not part of the claimed invention.
Note that in Table 1, MGS-F50V (glycerin number: 1, stearic acid number: 1, HLB=3.5, manufactured by Nikko Chemicals Co., Ltd.) as glyceryl monostearate and DECAGLYN 1-L (glycerin number: 10, lauric acid number: 1, HLB=15.5, manufactured by Nikko Chemicals Co., Ltd.) as decaglyceryl monolaurate were used respectively. As glycerin, a product of Kao Corporation was used.

[Comparative Example 3]

Powder composition 9 was obtained by preparation of each phase composition, emulsification and spray dry in the same manner as Example 1, except that the kind and content of the oil phase components and the aqueous phase components were changed as shown in Table 1 and the oil phase composition was prepared by only mixing the oil phase component without heat.

[Comparative Example 4]

Powder composition 10 was obtained by preparation of each phase composition, emulsification and spray dry in the same manner as Example 1, except that the heat treatment at the time of preparing the oil phase composition was performed at 70°C for 30 minutes.

[Table 1]

| | | Example 1 | Example 2 | Ref. Example 3* | Ref. Example 4* | Example 5 | Ref. Example 6* | Comp. Example 1 | Comp. Example 2 | Comp. Example 3 | Comp. Example 4 |
|---|---|---|---|---|---|---|---|---|---|---|---|
| Oil phase | Lycopene paste (lycopene content 18%) (g) | 8.9 | 5.9 | 8.9 | 8.9 | 8.9 | 8.9 | 8.9 | 8.9 | 8.9 | 8.9 |
| | Diglyceryl monostearate (g) | 0.9 | 0.6 | | 0.9 | 0.9 | | 0.0 | 0.0 | 0.0 | 0.9 |
| | Hexaglyceryl pentastearate (g) | | | 0.9 | | | | | | | |
| | Glyceryl monostearate (g) | | | | | | 0.9 | | | | |
| | Decaglyceryl monolaurate (g) | | | | | | | | 0.9 | | |
| | Tri(caprylic acid/capric acid)glyceryl (g) | | 2.9 | | | | 4.7 | 0.9 | | 0.9 | |
| | Calcium ascorbate (g) | 3.5 | 2.3 | 3.5 | 0.0 | | 7.1 | 3.5 | 3.5 | 0.0 | 3.5 |
| | Magnesium ascorbyl phosphate (g) | | | | | 7.1 | | | | | |
| | Mix tocopherol (g) | 1.3 | 0.9 | 1.3 | 1.3 | 1.3 | 1.3 | 1.3 | 1.3 | 1.3 | 1.3 |
| Aqueous phase | Pure water (g) | 246.9 | 244.4 | 246.9 | 246.9 | 246.9 | 246.9 | 246.9 | 246.9 | 246.9 | 246.9 |
| | Sucrose laurate (g) | 11.1 | 37.0 | 11.1 | 11.1 | 11.1 | 12.8 | 11.1 | 11.1 | 6.1 | 11.1 |
| | Decaglyceryl laurate (g) | | | | | | 4.3 | | | 2.0 | |
| | lecithin (g) | 1.8 | 6.0 | 1.8 | 1.8 | 1.8 | 2.8 | 1.8 | 1.8 | 1.8 | 1.8 |
| | Inulin (g) | 25.6 | 0.0 | 25.6 | 29.1 | 22.0 | 0.0 | 25.6 | 25.6 | 32.1 | 25.6 |
| | Glycerin (g) | | | | | | 10.3 | | | | |

(continued)

| | Example 1 | Example 2 | Ref. Example 3* | Ref. Example 4* | Example 5 | Ref. Example 6* | Comp. Example 1 | Comp. Example 2 | Comp. Example 3 | Comp. Example 4 |
|---|---|---|---|---|---|---|---|---|---|---|
| Lycopene concentration (emulsion) | 0.53% | 0.35% | 0.53% | 0.53% | 0.53% | 0.53% | 0.5% | 0.5% | 0.5% | 0.53% |
| Lycopene concentration (powder) | 3.0% | 2.00% | 3% | 3.0% | 3.0% | - | 3.0% | 3.0% | 3.0% | 3.0% |

*Reference Example - not part of the claimed invention

<Evaluation>

Evaluation of the emulsion before the drying process and of the obtained powder composition was conducted as follows. Further, evaluation of a sample consisting of LYCOPENE 18 was conducted as Comparative Example 5. These evaluation results are shown in Table 1.

(1) DSC endothermic peak temperature

The endothermic temperature and the exothermic temperature were measured by one cycle of temperature increase and temperature drop (15°C/min) in a range of from 30°C to 200°C using a DSC Q 2000 (TA Instruments Japan, Inc.) with the emulsified composition freeze-dried to remove any moisture therefrom, and the powder composition used in the powder state.

(2) Evaluation of crystal by observation under a polarizing microscope

Visual observation was conducted using a PCLIPSE LV100POL (Nicon Corporation) such that the emulsified composition was observed as the emulsion, while the powder composition was dissolved in water for observation. The evaluation results of the visual observation were classified as follows. Note that an evaluation of A or B corresponds to at least 90% by mass of the crystalline carotenoid being non-crystalline.

Visual observation

A: Almost no crystal derived from lycopene is found.
B~A: A slight presence of crystals derived from lycopene is found.
B: Crystals derived from lycopene are scattered, but to a minor extent.
C: Crystals derived from lycopene are present throughout the observed image.

(3) Average particle diameter

The average particle diameter of dispersion particles in an emulsion obtained from the aqueous phase components and the oil phase components was measured by diluting the emulsion 20-fold with pure water, while the average particle diameter of the powder dispersion was measured by diluting it with pure water so that the solid concentration became 1%, and by reading the respective values of d=50 at 25°C as the average particle diameter, using a PARTICLE SIZE ANALYZER PFARE-1000 (OTSUKA ELECTRONICS CO., LTD.).

(4) Lycopene residual rate

In the case of the emulsion or the powder composition in Examples 1, 2 and 5, Reference Exampels 3, 4 and 6 and Comparative Examples 1 to 4, in order to effect a lycopene concentration of 0.005 % by volume, the emulsion was diluted 1062-fold with acetone so as to sufficiently dissolve it. Further, in order to effect a lycopene concentration of 0.005 % by volume in the same manner as above, the powder composition was diluted 5.65-fold with pure water to sufficiently dissolve it, and then diluted 1062-fold with acetone to sufficiently dissolve it. Next, after filtration through a filter of 0.45 $\mu$m, the absorbance of the filtrate at the maximum peak wavelength (465 nm to475 nm) was measured using a spectrophotometer V-630 (manufactured by JASCO Corporation).
In addition, in the case of Example 2, the residual rate was measured in the same manner as Examples 1 and 5, Reference Examples 3, 4 and 6 and Comparative Examples 1 to 4, except that, in order to effect a lycopene concentration of 0.005 % by volume, the emulsion was diluted 708-fold with acetone to sufficiently dissolve it, while the powder composition was diluted 5.65-fold with pure water to sufficiently dissolve it, and then diluted 708-fold with acetone to sufficiently dissolve it.
Evaluation was conducted by diluting LYCOPENE 18 with acetone in order to effect a lycopene concentration of 0.005 % by volume, and measuring the absorbance at the peak wavelength in the same manner as above, and the percentage when the absorbance of this lycopene was taken as 100% was defined as the lycopene residual rate of the respective compositions.

(4) Dynamic absorbing property

The emulsion and the powder composition in Examples 1 and 5, Reference Examples 3, 4 and 6 and Comparative Examples 1 to 5 (Comparative Example 5 is a dilution in which the lycopene concentration is adjusted to 2 mg/ml with COCONAD MT) were diluted in order to effect a lycopene concentration of 2 mg/ml. Then, the dilution was orally administered (each group: n=4) to a non-fasted 6 week-old male rat at a dose of 10 ml/kg and then, after each of 1, 2, 3, 4, 6, 8 and 24 hours, 0.4 ml of blood was collected. A clear supernatant was separated from the collected blood by centrifugation and 0.1 ml of blood plasma was extracted only from the clear supernatant. The blood plasma was dissolved in acetone, and then hexane was added, and then the mixture was left to stand. The resultant clear supernatant was recovered. The recovered clear supernatant was dried by solidification, and then the solid was re-dissolved in chloroform= 1/1 (vl/vl), and then the content of lycopene was measured by HPLC.

The relationship between the time from administration to collection of blood and the lycopene concentration was illustrated graphically. Then, an AUC (area under the blood concentration-time curve) over a period of 8 hours from administration with respect to each administered composition was measured. This measured value was defined as the value of the dynamic absorbing property. The results are shown in Table 2. A higher numerical number is rated as a higher concentration of active substances in the blood.

[Table 2]

| | Example 1 | Example 2 | Ref. Example 3* | Ref. Example 4* | Example 5 | Ref. Example 6* | Comp. Example 1 | Comp. Example 2 | Comp. Example 3 | Comp. Example 4 | Comp. Example 5 |
|---|---|---|---|---|---|---|---|---|---|---|---|
| DSC absorption peak temperature | none | none | none | none | none | none | 153°C | 154°C | 153°C | 154°C | 153°C |
| Observation under a polarizing microscope | A | A | B~C | A | A | A | C | C | C | C | C |
| Average particle diameter (nm) | | | | | | | | | | | |
| Emulsion | 125 | 212 | 120 | 121 | 123 | 52 | 113 | 122 | 145 | 1μm≤ | - |
| Powder composition | 140 | - | 131 | 129 | 138 | - | 121 | 136 | 175 | 1μm≤ | - |
| Lycopene residual rate | 96% | 98% | 97% | 30% | 92% | 99% | 97% | 99% | 99% | 100% | |
| AUC (ng*8h/ml) | 5390 | 8730 | - | - | - | - | - | - | 830 | - | 540 |
| * Reference Example - not part of the claimed invention | | | | | | | | | | | |

EP 2 574 337 B1

As shown in Tables 1 and 2, the carotenoid-containing compositions of Examples 1, 2 and 5 and Reference Examples 3, 4 and 6, in which an oil phase composition was prepared by heat-treating LYCOPENE 18 together with a polyglyceryl fatty acid ester having from 1 to 6 glycerin units and from 1 to 5 fatty acid units at a temperature higher than the melting point of the LYCOPENE 18 were compositions in which crystallization was suppressed, based on the fact that a DSC endothermic peak was not found, regardless of whether the compositions were in emulsion form or powder form.

As shown in Table 2, a DSC endothermic peak derived from crystals is not found in any of the carotenoid-containing compositions of the Examples and Reference Examples. According to the understanding of the present inventors, in a case where 10% by mass or greater of the crystalline carotenoid is contained, the DSC endothermic peak is observed. Accordingly, the results of observation under a polarizing microscope and the DSC endothermic peak temperature indicate that the content of crystal derived from lycopene contained in the carotenoid-containing composition of Reference Example 3 is greater than 0, but less than 10% by mass.

Further, the results of administration tests on rats demonstrate that all of the carotenoid-containing compositions of the Examples and Reference Examples show excellent lycopene absorbing properties, and are carotenoid-containing compositions that show high absorbance properties due to the suppression of lycopene crystallization.

Thus, according to the present invention, even though the composition contains a high-crystalline carotenoid, a carotenoid-containing composition in which crystallization is suppressed can be produced.

## Claims

1. A method of producing a carotenoid-containing composition, the method comprising:

   obtaining a carotenoid-containing oil phase composition by heating an oil phase component mixed liquid comprising a carotenoid component comprising at least lycopene, and diglyceryl monostearate , at a temperature of the melting point of the carotenoid component or higher; and
   obtaining an oil-in-water emulsified composition by emulsifying under pressure the carotenoid-containing oil phase composition with an aqueous-phase composition comprising an emulsifying agent;
   wherein the lycopene content is from 0.1 % by mass to 5% by mass with respect to the total mass of the solid content in the carotenoid-containing composition;
   wherein the total mass of diglyceryl monostearate is from 0.01 times to 10 times with respect to the total mass of lycopene;
   the method further comprising incorporating an antioxidant in the oil phase component mixed liquid before the heating, wherein the antioxidant is at least one selected from the group consisting of ascorbic acid, an ascorbic acid ester, and a salt thereof.

2. The method of producing a carotenoid-containing composition according to claim 1, wherein a total mass of the antioxidant is from 0.05 times to 50 times with respect to a total mass of the crystalline carotenoid.

3. The method of producing a carotenoid-containing composition according to any one of claims 1 to 2, wherein the carotenoid-containing composition further comprises at least one water-soluble encapsulating agent selected from a sugar polymer comprising a sugar unit comprising at least two fructose units or an oligomer comprising a sugar unit comprising at least two fructose units.

4. The method of producing a carotenoid-containing composition according to claim 3, wherein a total mass of the water-soluble encapsulating agent is from 0.5 times to 50 times with respect to a total mass of oil components comprising the carotenoid component.

5. The method of producing a carotenoid-containing composition according to any one of claims 1 to 4, wherein a total mass of the emulsifying agent contained in the aqueous phase composition is from 0.1 times to 10 times with respect to a total mass of oil components comprising the carotenoid component.

6. The method of producing a carotenoid-containing composition according to any one of claims 1 to 5, further comprising obtaining a powder composition by drying the oil-in-water emulsified composition.

7. The method of producing a carotenoid-containing composition according to any one of claims 1 to 6, wherein an average particle diameter of a re-dissolved oil-in-water emulsified composition that has been obtained by re-dissolving the oil-in-water emulsified composition or the powder composition is from 50 nm to 300 nm.

8. A carotenoid-containing composition obtained by the method as defined in claim 1, comprising: a carotenoid component comprising at least lycopene, at least 90% by mass of the lycopene being non-crystalline; and diglyceryl monostearate;

   wherein the lycopene content is from 0.1 % by mass to 5% by mass with respect to the total mass of the of the solid content in the carotenoid-containing composition; and

   wherein the total mass of diglyceryl monostearate is from 0.01 times to 10 times with respect to the total mass of lycopene.


**Patentansprüche**

1. Verfahren zur Herstellung einer Carotenoid-haltigen Zusammensetzung, wobei das Verfahren umfasst:

   Erhalten einer Carotenoid-haltigen Ölphasenzusammensetzung durch Erwärmen einer gemischten Ölphasenkomponenten-Flüssigkeit, umfassend eine Carotenoid-Komponente, die zumindest Lycopen umfasst, und Diglycerylmonostearat, bei einer Temperatur des Schmelzpunkts der Carotenoid-Komponente oder höher; und
   Erhalten einer emulgierten Öl-in-Wasser-Zusammensetzung durch Emulgieren der Carotenoid-haltigen Ölphasenzusammensetzung unter Druck mit einer wässrigen Phasenzusammensetzung, die einen Emulgator umfasst;
   worin der Lycopengehalt von 0,1 Masse-% bis 5 Masse-% beträgt, bezogen auf die Gesamtmasse der festen Bestandteile in der Carotenoid-haltigen Zusammensetzung;
   worin die Gesamtmasse von Diglycerylmonostearat vom 0,01-fachen bis 10-fachen im Hinblick auf die Gesamtmasse von Lycopen beträgt;
   wobei das Verfahren ferner das Einarbeiten eines Antioxidans in die gemischte Ölphasenkomponenten-Flüssigkeit vor dem Erwärmen umfasst, worin das Antioxidans zumindest eines ist, ausgewählt aus der Gruppe, bestehend aus Ascorbinsäure, einem Ascorbinsäureester und einem Salz hiervon.

2. Verfahren zur Herstellung einer Carotenoid-haltigen Zusammensetzung gemäß Anspruch 1, worin die Gesamtmasse des Antioxidans vom 0,05-fachen bis 50-fachen im Hinblick auf die Gesamtmasse des kristallinen Carotenoids beträgt.

3. Verfahren zur Herstellung einer Carotenoid-haltigen Zusammensetzung gemäß irgendeinem der Ansprüche 1 bis 2, worin die Carotenoid-haltige Zusammensetzung ferner zumindest ein wasserlösliches Einkapselungsmittel umfasst, ausgewählt aus einem Zuckerpolymer, das eine Zuckereinheit umfasst, die mindestens zwei Fruktoseeinheiten umfasst, oder einem Oligomer, das eine Zuckereinheit umfasst, die mindestens zwei Fruktoseeinheiten umfasst.

4. Verfahren zur Herstellung einer Carotenoid-haltigen Zusammensetzung gemäß Anspruch 3, worin die Gesamtmasse des wasserlöslichen Einkapselungsmittels vom 0,5-fachen bis zum 50-fachen im Hinblick auf die Gesamtmasse der Ölkomponenten, die die Carotenoid-Komponente umfassen, beträgt.

5. Verfahren zur Herstellung einer Carotenoid-haltigen Zusammensetzung gemäß irgendeinem der Ansprüche 1 bis 4, worin die Gesamtmasse des Emulgators, der in der wässrigen Phasenzusammensetzung enthalten ist, vom 0,1-fachen bis 10-fachen im Hinblick auf die Gesamtmasse der Ölkomponenten, die die Carotenoid-Komponente umfassen, beträgt.

6. Verfahren zur Herstellung einer Carotenoid-haltigen Zusammensetzung gemäß irgendeinem der Ansprüche 1 bis 5, ferner umfassend das Erhalten einer Pulverzusammensetzung durch Trocknen der emulgierten Öl-in-Wasser-Zusammensetzung.

7. Verfahren zur Herstellung einer Carotenoid-haltigen Zusammensetzung gemäß irgendeinem der Ansprüche 1 bis 6, worin der mittlere Partikeldurchmesser einer wieder aufgelösten emulgierten Öl-in-Wasser-Zusammensetzung, die durch Wiederauflösen der emulgierten Öl-in-Wasser-Zusammensetzung oder der Pulverzusammensetzung erhalten wird, von 50 nm bis 300 nm beträgt.

8. Carotenoid-haltige Zusammensetzung, erhalten durch das Verfahren gemäß Anspruch 1, umfassend: eine Carotenoid-Komponente, die zumindest Lycopen umfasst, worin mindestens 90 Masse-% des Lycopens nicht-kristallin ist; und Diglycerylmonostearat;
   worin der Lycopengehalt von 0,1 Masse-% bis 5 Masse-% beträgt, bezogen auf die Gesamtmasse der festen Bestandteile in der Carotenoid-haltigen Zusammensetzung; und
worin die Gesamtmasse von Diglycerylmonostearat vom 0,01-fachen mit 10-fachen, bezogen auf die Gesamtmasse von Lycopen, beträgt.

**Revendications**

1. Procédé de production d'une composition à base de caroténoïde, le procédé comprenant:

   l'obtention d'une composition huileuse à base de caroténoïde en chauffant un mélange de composants huileux comprenant un composant de caroténoïde comprenant au moins du lycopène et du monostéarate de diglycéryle, à une température du point de fusion du composant de caroténoïde ou plus ; et
   l'obtention d'une composition émulsifiée huile dans eau en émulsifiant sous pression la composition huileuse contenant du caroténoïde avec une composition à phase aqueuse comprenant un agent émulsifiant ;
   dans lequel la teneur en lycopène va de 0,1% en masse à 5 % en masse par rapport à la masse totale de la teneur solide de la composition à base de caroténoïde ;
   dans lequel la masse totale de monostéarate de diglycéryle fait de 0,01 fois à 10 fois par rapport à la masse totale de lycopène ;
   le procédé comprenant en outre l'incorporation d'un antioxydant dans le mélange de composants huileux avant le chauffage, dans lequel l'antioxydant est au moins un sélectionné dans le groupe constitué d'acide ascorbique, d'un ester d'acide ascorbique et d'un sèl de ceux-ci.

2. Procédé de production d'une composition à base de caroténoïde selon la revendication 1, dans lequel une masse totale de l'antioxydant fait de 0,05 fois à 50 fois par rapport à une masse totale du caroténoïde cristallin.

3. Procédé de production d'une composition à base de caroténoïde selon l'une quelconque des revendications 1 à 2, dans lequel la composition à base de caroténoïde comprend en outre au moins un agent d'encapsulation soluble dans l'eau sélectionné à partir d'un polymère de sucre comprenant une unité de sucre comprenant au moins deux unités de fructose ou un oligomère comprenant une unité de sucre comprenant au moins deux unités de fructose.

4. Procédé de production d'une composition à base de caroténoïde selon la revendication 3, dans lequel une masse totale de l'agent d'encapsulation soluble dans l'eau fait de 0,5 fois à 50 fois par rapport à une masse totale de composants d'huile comprenant le composant de caroténoïde.

5. Procédé de production d'une composition à base de caroténoïde selon l'une quelconque des revendications 1 à 4, dans lequel une masse totale de l'agent émulsifiant contenu dans la composition à phase aqueuse fait de 0,1 fois à 10 fois par rapport à une masse totale de composants d'huile comprenant le composant de caroténoïde.

6. Procédé de production d'une composition à base de caroténoïde selon l'une quelconque des revendications 1 à 5, comprenant en outre l'obtention d'une composition de poudre en séchant la composition émulsifiée huile dans eau.

7. Procédé de production d'une composition à base de caroténoïde selon l'une quelconque des revendications 1 à 6, dans lequel un diamètre de particule moyen d'une composition émulsifiée huile dans eau re-dissoute qui a été obtenue par la redissolution de la composition émulsifiée huile dans eau ou de la composition de poudre fait de 50 nm à 300 nm.

8. Composition à base de caroténoïde obtenue par le procédé tel que défini dans la revendication 1, comprenant : un composant de caroténoïde comprenant au moins du lycopène, au moins 90 % en masse du lycopène étant non cristallin ; et du monostéarate de diglycéryle ;
   dans laquelle la teneur en lycopène fait de 0,1% en masse à 5 % en masse par rapport à la masse totale de la teneur solide dans la composition contenant du caroténoïde ; et
   dans laquelle la masse totale de monostéarate de diglycéryle fait de 0,01 fois à 10 fois par rapport à la masse totale de lycopène.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008013751 A
- JP 10120933 A
- JP 9157159 A

**Non-patent literature cited in the description**

- Hand book of Hydrocolloides. CRC Press, 2000, 397-403